# EUROPEAN PATENT APPLICATION

(11) **EP 1 541 125 A1**
(43) Date of publication of application: **15.06.2005**
(21) Application number: 04101355.8
(22) Date of filing: 01.04.2004
(51) Int. Cl.: A61K 7/48, A61K 9/06, A61K 31/00, A61K 31/215

(54) **Spreadable compositions for topical use, a process of making same and uses thereof**

(30) Priority: 03.12.2003 US 526281 P; 03.12.2003 US 526282 P; 08.03.2004 US 794184; 08.03.2004 US 795116
(71) Applicant: AGIS INDUSTRIES (1983) LTD, Bnei-Brak 51200 (IL)
(72) Inventor: Asculai, Elion, 85338, Lehavim (IL); Zeevi, Amira, 84965, Omer (IL); Cherkez, Stephen, 38900, Caesarea (IL); Schneider, Benjamin, 76464, Rehovot (IL)
(74) Representative: Schmitz, Jean-Marie

(57) **Abstract**

A process of preparing a spreadable composition containing a hard fat, spreadable compositions prepared thereby and methods of using spreadable pharmaceutical, cosmetic and cosmeceutical compositions are provided.

## Description

### FIELD AND BACKGROUND OF THE INVENTION

The present invention relates to spreadable compositions for topical application, a process of manufacturing such compositions, and uses of such compositions.

Topical pharmaceutical and cosmetic compositions are used for or to deliver, for example, skin treating agents, skin conditioning agents, water proofing agents, sunscreens, lip balms, wound dressings, hair pomades. Regardless of the specific use, topical compositions are preferably formulated so as to remain on an applied surface for an extended period of time, so as to be effective. Topical compositions preferably do not irritate membranes such as skin, eyes and mucous membranes. A topical composition preferably has a good "cosmetic feeling" that is when used subjectively feel good to a user: the composition must feel neither too watery nor too greasy.

Ointment preparations are presently known to be a more efficient dosage form than other semi-solid topical preparations (i.e. creams, lotions, pastes and gels). The greater efficiency is related mainly to the nature of the ointment base: the base of ointments has an occlusion effect that reduces dehydration of a surface on which it is applied and also leads to an increased permeation of any active substances.

As used herein throughout and is widely acceptable in the related art, the term "base" in the field of topical applications describes the entirety of non-active components in a formulation, which typically determines the final form and properties of the formulation. Hence, for example, ointment bases provide for the occlusive effect described above, and their physical characteristics (e.g., spreadability, viscosity, greasiness, stiffness, stickiness) determine the "cosmetic feeling" of the ointment.

Four types of ointment bases are presently listed in the United States Pharmacopoeia: hydrocarbon bases (oleaginous, i.e. white petrolatum); anhydrous absorption bases (e.g., anhydrous lanolin); water-in-oil emulsion type absorption bases (e.g., lanolin); and water-removable bases (oil-in-water emulsion type).

The main drawbacks of prior art ointment preparations include a greasy feel, stiffness and spreading difficulty of the applied ointment. Thus, for example, water-free preparations are hard to wash and fail to melt on the skin and therefore, must be rubbed over the designated area in order to achieve a homogenous thin layer when applied. These characteristics are highly problematic especially in cases when a preparation is used for application to dry and/or wounded skin or in the treatment of diseases such as psoriasis, impetigo and the like. In such cases, it is hard and unpleasant to apply a greasy, stiff, non-washable ointment preparation on the infected area, and hence the efficiency and usage convenience of the preparation are severely limited.

One way to overcome the above limitations is the use of ointments that are based on poly(alkylene)glycols, poly(alkoxy substituted alkylene)glycols and derivatives thereof (collectively referred to herein and in the art as PAG or PAGs). However, ointments based on PAGs suffer from several drawbacks that limit the use and consumer acceptance of such ointments.

For example, topically administered PAGs typically cause a stinging feeling, urticaria and may further cause delayed allergic reactions (see, *The Handbook of Pharmaceutical Excipients 3*^{*rd*} *ed.* 2002 p. 396). The most serious adverse effects associated with PAGs are hyperosmolarity, metabolic acidosis, and renal failure following the topical use by bum patients ("Topical PEG in burn ointment" FDA Drug Bull. 1982, 12: 25).

Therefore, despite promising results recorded when using an ointment comprising Mupirocin and PEG to treat bum wounds in rats, PEG containing topical preparations are contraindicated for patients with renal failure, extensive burns, open wounds and damaged skin (Rode *et al.* J. Antimicrob. Chemother. 1988, 21: 589-595; Kaczmarski J. Antimicrob. Chemother. 1988, 22: 771-776; and Rode *et al.* J. Antimicrob. Chemother, 1989, 24: 78-79). As a result, compositions containing high percentages of PAGs are required to carry a warning label. Products containing none or only low percentages of PAGs are not so labeled.

A further disadvantage of compositions comprising PAGs is that when applied to the skin, these compositions leave an unpleasant sticky feeling on an applied area, reducing patient compliance. An even further disadvantage of PAG based compositions is that they cannot be applied to mucous membranes.

In the art, hard fats are known as being nontoxic, nonirritating, can be formulated to melt on contact with skin, are compatible with a variety of active pharmaceutical ingredient, and have been approved for pharmaceutical uses. Hard fats have a substantially solid (butter-like) consistency at room temperature and are therefore well known for use in the preparation of rectal and vaginal suppositories (see The Science and Practice of Pharmacy, Remington, 19^{th} Ed. pp. 1592).

Due to their solid, non-spreadable nature, hard fats are not suitable and therefore not generally known for use in the preparation of spreadable compositions (e.g., ointments) for topical application. In the very few cases where hard fats are found in commercial topical preparations (*e.g.*, Advantan® Cream, Sobering AG, Berlin, Federal Republic of Germany), they are used in low concentrations (*Handbook of Pharmaceutical Excipients,* 3rd Ed., pp. 550).

U.S. Patent No. 6,365,635, U.S. Patent Application No. 20020142040 and EP Patent Application No. 1112750, all by Numora et al., teach antibacterial topical compositions that include an antibacterial active ingredient and a non-aqueous hydrophobic base. Such compositions, according to teachings of these references, are prepared by mixing the composition components, preferably by heating, and cooling the resulting mixture. While hard fats are recited in these references as one of the optional non-aqueous hydrophobic bases, compositions containing hard fat are not exemplified. As is discussed hereinabove, it is reasonable to assume that such compositions would not result in a spreadable preparation.

An exception is found in U.S. Patent No. 6,013,657 where a composition that comprises Mupirocin, a carrier comprising oleyl alcohol and/or castor oil and optionally a hard fat ointment base, which has a spreadable consistency, is taught. The composition of U.S. Patent No. 6,013,657 has numerous advantages including non-toxicity; suitability for application to eyes, mucous membranes, open wounds and bum wounds; skin compatibility and spreadability; and superior skin penetration properties (Hermsdorf Cosmetic and toiletries 1980, 95: 61-63).

While practicing the teachings of U.S. Patent No. 6,013,657, the present inventors have uncovered an improved process for preparing the composition disclosed therein, which can be efficiently utilized for preparing spreadable compositions in general. The novel process is characterized by high batch-to-batch viscosity reproducibility and the resulting composition is characterized by physical properties that are highly suitable for ointment preparations and is devoid of the limitations described above.

### SUMMARY OF THE INVENTION

According to the teachings of the present invention there is provided a process of preparing a spreadable composition for topical application, the process comprising: providing a mixture including at least one hard fat; heating the mixture to a first temperature, the first temperature being higher than a congelation temperature of the mixture; cooling the mixture to a second temperature; re-heating the mixture to a third temperature, the third temperature being higher than the congelation temperature of the mixture; and re-cooling the mixture to an ambient temperature, thereby obtaining the spreadable composition of the present invention. Preferably, the heating, the cooling and/or the re-heating are performed while stirring the mixture.

According to a preferred embodiment of the present invention, the second temperature is lower than the congelation temperature of the mixture.

According to an embodiment of the present invention, the first temperature ranges between about 40 °C and about 100 °C, more preferably between about 50 °C and about 80 °C.

According to an embodiment of the present invention, the second temperature ranges between about 10 °C and about 30 °C, more preferably between about 18 °C and about 28 °C.

According to an embodiment of the present invention, the third temperature ranges between about 30 °C and about 40 °C, more preferably between about 30 °C and about 35 °C.

According to an embodiment of the present invention, the mixture is maintained at the third temperature for a period of time that ranges between about 180 minutes and about 600 minutes, preferably for about 300 minutes.

According to a feature of the present invention, subsequent to the re-heating and prior to the re-cooling, a receptacle is filled with the mixture. Preferably, during the filling the temperature of the mixture is substantially maintained at a fourth temperature. The fourth temperature preferably ranges between about 20 °C and about 40 °C, more preferably between about 30 °C and about 35 °C.

According to a feature of the present invention, the resulting spreadable composition has a viscosity that ranges between about 2 Poise and about 2500 Poise, and preferably between about 2 Poise and about 2000 Poise, at a temperature of about 20 °C.

According to a feature of the present invention, the concentration of the at least one hard fat ranges between about 20 weight percentages and about 99 weight percentages, preferably between about 20 weight percentages and about 80 weight percentages, and even more preferably between about 50 weight percentages and about 80 weight percentages, of the total weight of the mixture (and consequently the spreadable composition of the present invention).

According to a feature of the present invention, the at least one hard fat comprises a triglyceride, preferably a mixed triester of glycerin with caprylic, capric and stearic acids.

In a preferred embodiment of the present invention, added to the mixture (and consequently a component of the spreadable composition of the present invention) is a pharmaceutically acceptable carrier.

According to an embodiment of the present invention, the pharmaceutically acceptable carrier comprises castor oil and/or oleyl alcohol.

According to a further embodiment of the present invention, the pharmaceutically acceptable carrier is selected from the group consisting of water, a glycol, a polyalkylene glycol, an ester, an amide, a protein hydrolysate, an alkylated protein hydrolysate, a lanolin or a derivative thereof, a monohydric alcohol, a polyhydric alcohol, an ether, a carbowax, a polyoxyethylene glycerol, a polyoxyethylene sorbitol, a stearoyl diacetin and any combination thereof.

According to one embodiment of the present invention, the mixture (and consequently the spreadable composition of the present invention) is substantially devoid of a PAG (a general term used herein to describe (poly(alkylene)glycols, poly(alkoxy substituted alkylene)glycols and derivatives thereof).

According to a different embodiment of the present invention, added to the mixture (and consequently a component of the spreadable composition of the present invention) comprises at least one PAG. Preferably the concentration of the PAG is less than about 20 weight percentages, more preferably less than about 10 weight percentages, and even more preferably less than about 1 weight percentage of the total weight of the mixture.

According to an embodiment of the present invention, added to the mixture (and consequently a component of the spreadable composition of the present invention) is a solvent. Preferably, such a solvent is selected from the group consisting of ethanol, n-propanol, isopropanol and mixtures thereof.

According to an embodiment of the present invention, added to the mixture (and consequently the spreadable composition of the present invention) is propylene glycol stearate.

According to an embodiment of the present invention, added to the mixture (and consequently a component of the spreadable composition of the present invention) is at least one additional component. Such an additional component is selected from a group including but not limited to an anti-dandruff agent, an anti-irritant, an anti-oxidant, an antiperspirant agent, a buffering agent, a bulking agent, a chelating agent, a colorant, a deodorant, a dermatological active ingredient, a diluent, an emollient, an emulsifier, a hair conditioner, a humectant, an occlusive agent, oil, a penetration enhancer, a skin penetration enhancer, a perfuming agent, a permeation enhancer, a pH adjusting agent, a preservative, a protectant, a softener, a solubilizer, a stearically stabilizing substance, a sunscreening agent, a sun blocking agent, a sunless tanning agent, a surfactant and a vitamin.

According to an embodiment of the present invention, added to the mixture (and consequently a component of the spreadable composition of the present invention) is at least one pharmaceutically, cosmetically and cosmeceutically active ingredient. Such an active ingredient include, without limitation, an antibiotic agent, an antimicrobial agent, an anti-acne agent, an antibacterial agent, an antifungal agent, an antiviral agent, an anti-inflammatory agent, an anesthetic agent, an antipruriginous agent, an antiprotozoal agent, an anti-oxidant, a chemotherapeutic agent, an antidepressant agent, a hormone, an antidandruff agent, a sunscreening agent, a sun blocking agent, a sunless tanning agent, a dye, a deodorant, a hair conditioning agent and a cleansing agent.

As stated above, according to the teachings of the present invention there is also provided a spreadable composition, preferably substantially prepared according to the process of the present invention, as described herein.

According to a feature of the present invention, the composition is packaged in a packaging material and identified in print, in or on the packaging material, for use for a need selected from the group consisting of curing a condition, treating a condition, preventing a condition, treating symptoms of a condition, curing symptoms of a condition, ameliorating symptoms of a condition, treating effects of a condition, ameliorating effects of a condition, and preventing results of a condition.

According to a feature of the present invention, and depending on the components making up a composition of the present invention, the condition can be an eye disorder, an ear disorder, a mucosal membrane disorder or a skin and/or scalp disease or disorder such as, but not limited to, actinic keratoses, eczema, dermatitis, BCC (basal cell carcinoma), superficial BCC, Bowen's disease, chronic superficial scaly, dermatosis, parapsoriasis, cradle cap, cutaneous T-cell lymphoma, Darier's disease, disseminated superficial, actinic porokeratosis, dry skin, erythrasma, exfoliative keratolysis, an infection, a fungal infection, Grover's disease, ichthyosis, juvenile plantar dermatosis, lichen striatus, lupus erythematosus, pellagra, pityriasis alba, pityriasis lichenoides, pityriasis rosea, pityriasis rubra pilaris, pityriasis versicolor, psoriasis, Reiter's syndrome, seborrhoeic dermatitis, a tinea infection, impetigo, a wound and a burn.

According to the teachings of the present invention there is also provided a method of treatment comprising administering an effective amount of a spreadable composition according to the present invention, which comprises one or more pharmaceutically, cosmetically or cosmeceutically active ingredient(s) to a mammal (human or non-human) in need thereof.

In one embodiment of the present invention the administration is preferably topical administration to one or more affected area(s) of the mammal, whereas the affected areas are preferably an ear, an eye, a skin a scalp and/or a mucosal membrane. In another embodiment, the administration can be effected buccally, dermally, intranasally, lingually, mucosally, rectally, sublingually, topically, urethrally and vaginally.

The need for which a spreadable composition of the present invention is used is generally a need arising from a condition in which treatment by the pharmaceutically active ingredient is beneficial. Depending on the components making up a composition of the present invention, such a condition includes but is not limited to an eye disorder, an ear disorder, a mucosal membrane disorder and a skin and/or scalp disorder or disease, as described hereinabove.

The present invention successfully addresses the shortcomings of the presently known configurations by providing an improved process of preparing a spreadable composition (e.g., a spreadable pharmaceutical composition) which is based on hard fats, which results in an efficient and usage convenient composition that can optionally and beneficially be used in various pharmaceutical, cosmetic and cosmeceutical applications.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### DESCRIPTION OF THE PREFERRED EMBODIMENTS

The present invention is of a process for making a spreadable composition that is substantially based on hard fats, in which a spreadable composition characterized by high suitability for topical application is obtained. The present invention is therefore also of a spreadable composition, particularly a spreadable composition made according to the process of the present invention, and of a method of treatment using such a spreadable composition containing a pharmaceutically, cosmetically and/or cosmeceutically active ingredient.

The principles and operation of the processes, compositions and methods of the present invention may be better understood with reference to the description and examples hereinbelow.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

The present inventors have now surprisingly uncovered a novel process in which a spreadable composition comprising a hard fat, which is highly suitable for topical application, is obtained. The process of the present invention reproducibly produces a composition that is occlusive, creamy, non-greasy, smooth and easy to spread, melts on the skin and needs not be rubbed over an area to achieve a homogenously thin layer. As is discussed hereinabove, such characteristics render a topical composition highly advantageous.

As used herein, the term "spreadable" means that the composition can be spread over an area.

The term "creamy" is used to mean that the composition has application characteristics similar to a cream.

The term "occlusive" is used to mean that the composition occludes the area onto which it is applied.

The term "non-greasy" is used to mean that the composition is devoid of greasiness, or, in other words, that no greasy feeling accompanies the application of the composition.

The term "smooth" is used to mean that the composition is characterized by a "creamy like" feeling when applied.

The phrase "easy to spread" is used to mean that the composition needs not be rubbed over an area to achieve a homogenously thin layer.

The properties described hereinabove are mainly attributed to the unique property of hard fat based compositions, which tend to melt upon contacting a body area.

As used herein, the term "active ingredient" or "pharmaceutically, cosmetically or cosmeceutically active ingredient" is a component of a composition that has a pharmaceutical, cosmetic or cosmeceutical activity.

As used herein the term "topical application" describes application onto a biological surface, e.g., an affected skin or membrane. Hence, the phrase "a composition for topical application" describes a composition that is applied, preferably by spreading, on, for example, skin, eye, ear, scalp, hair or membrane of a subject.

As used herein, the term "process" and the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

The process of the present invention for preparing a spreadable composition comprises: providing a mixture including at least one hard fat; heating the mixture to a first temperature, which being higher than the congelation temperature of the mixture; cooling the mixture to a second temperature; (the second temperature preferably lower than the congelation temperature of the mixture); re-heating the mixture to a third temperature, which is higher than the congelation temperature of the mixture; and re-cooling the mixture to an ambient temperature. Preferably, the heating, the cooling and/or the re-heating are performed while the mixture is stirred.

As used herein, the term "congelation temperature" refers to the highest temperature at which the mixture congeals, that is that the mixture starts to become solid rather than liquid.

By cooling or re-cooling is meant herein that the temperature of the mixture is reduced either actively (by cooling) or passively (by letting the mixture cool down). Preferably the mixture is mixed during the cooling process.

According to a preferred embodiment, the first temperature ranges between about 30 °C and about 100 °C, preferably between about 40 °C and about 100 °C, more preferably between about 40 °C and about 90 °C, more preferably between about 50 °C and about 90 °C, more preferably between about 50 °C and about 80 °C, and even more preferably between about 60 °C and about 80 °C.

As used herein, the term "about" refers to ± 10 %.

According to another preferred embodiment, during the heating step, the mixture is maintained at the first temperature for a time period that ranges between about 10 minutes and about 240 minutes, more preferably between about 15 minutes and about 120 minutes, more preferably between about 15 minutes and about 60 minutes.

After the heating step, the mixture is cooled to a second temperature that is preferably lower than a congelation temperature of the mixture.

As a typical congelation temperature of the hard fat-based mixture of the present invention is lower than about 30 °C, according to a preferred embodiment, the second temperature ranges between about 10 °C and about 30 °C, more preferably between about 15 °C and about 30 °C, more preferably between about 18 °C and about 28°C, and most preferably between about 20 °C and about 25 °C.

Once the mixture reaches the second temperature, it is re-heated to a third temperature, which again, is higher than the congelation temperature of the mixture. According to a preferred embodiment, the third temperature ranges between about 30 °C and about 40 °C, more preferably between about 30 °C and about 35 °C.

In a preferred embodiment, the mixture is maintained at the third temperature for a period of time that ranges between about 180 minutes and about 600 minutes, more preferably between about 240 minutes and about 480 minutes, preferably about 300 minutes.

In another preferred embodiment of the present invention, subsequent to re-heating and prior to re-cooling the mixture, a receptacle is filled with the mixture. Preferably, during the filling of the receptacle, the temperature of the mixture is substantially maintained at a fourth temperature that ranges between about 20 °C and about 40 °C, or more preferably between about 30 °C and about 35 °C.

The receptacle can be, for example, a tube (such as a squeeze tube), a jar or a bottle (for example with a pump dispenser).

The process of the present invention is preferably performed in greater than laboratory scale and has certain advantages when so performed. Thus the process of the present invention is preferably performed when the volume of the mixture is greater than 1 liter, more preferably greater than 2 liter, even more preferably greater than 3 liter, even more preferably greater than 4 liter, even more preferably greater than 5 liter, even more preferably greater than 6 liter, even more preferably greater than 7 liter, even more preferably greater than 8 liter, even more preferably greater than 9 liter and even more preferably greater than 10 liter.

According to a feature of the present invention, the spreadable pharmaceutical composition thus produced has a viscosity that ranges between about 2 Poise and about 2500 Poise at a temperature of about 20 °C, more preferably between about 2 Poise and about 2000 Poise. As is demonstrated in the Examples section that follows, the viscosity of the composition remains substantially unchanged for at least 6 months.

The advantageous properties of the spreadable composition produced by the process of the present invention are mainly attributed to the presence of the hard fat(s). Without being bound to any particular theory, it is assumed that these properties are obtained by re-altering the mixture temperature as described hereinabove.

The term "hard fat" is used herein as understood in the United States Pharmacopoeia and substantially as defined in The Handbook of Pharmaceutical Excipients, 3^{rd} Edition, page 550. As described therein, hard fats typically consist mainly of mixtures of the triglyceride esters of the higher saturated fatty acids (C₈H₁₇COOH through C₁₈H₃₇COOH). Hard fats optionally include varying proportions of mono- and diglycerides. For the present invention the term "hard fat" also includes substantially pure triglyceride esters. A preferred hard fat comprises a triglyceride, preferably a mixed triester of glycerin with caprylic, capric and stearic acids.

According to a feature of the present invention, the concentration of the at least one hard fat is greater than 20 weight percentages of the total weight of the mixture (and consequently the spreadable composition of the present invention), more preferably greater than 30 weight percentages, more preferably greater than 40 weight percentages, more preferably greater than 50 weight percentages, more preferably greater than 60 weight percentages, more preferably greater than 70 weight percentages, more preferably greater than 80 weight percentages, and in some cases it is greater than 90 weight percentages and up to 100 weight percentages, such that the composition comprises only the hard fat(s). Such a composition can serve as a base, as is defined hereinabove, for various pharmaceutical, cosmetic and cosmeceutical preparation.

Hence, the concentration of the at least one hard fat preferably ranges between about 20 weight percentages and about 100 weight percentages, more preferably between about 20 weight percentages and about 80 weight percentages, and even more preferably between about 50 weight percentages and about 80 weight percentages, of the total weight of the mixture (and consequently the spreadable composition of the present invention).

In a preferred embodiment of the present invention, the mixture (and consequently the spreadable composition of the present invention) further comprises a carrier. Preferably, and according to the intended use of the final composition, the carrier is a pharmaceutically, cosmetically or cosmeceutically acceptable carrier.

As used herein, the term "pharmaceutically, cosmetically or cosmeceutically acceptable carrier'' describes a carrier or a diluent that does not cause significant irritation to an organism and does not abrogate the biological activity and properties of the applied active ingredient.

As used herein and is widely acceptable in the related art, a carrier is typically used as a vehicle, which solubilizes and delivers the active ingredient to the treated/desired area(s).

According to an embodiment of the present invention, the carrier comprises castor oil and/or oleyl alcohol. According to a further embodiment of the present invention, other suitable carrier for use in the context of the present invention include, without limitation, water, and other liquid carriers such as alcohols, glycols, polyalkylene glycols, esters, amides, protein hydrolysates, alkylated protein hydrolysates, lanolin and lanolin derivatives, and like materials commonly employed in cosmetic and medicinal compositions.

Other suitable carriers include for example alcohols, including both monohydric and polyhydric alcohols, e.g., ethanol, isopropanol, glycerol, sorbitol, 2-methoxyethanol, diethyleneglycol, ethylene glycol, hexyleneglycol, mannitol, and propylene glycol; ethers such as diethyl or dipropyl ether; polyethylene glycols and methoxypolyoxyethylenes (carbowaxes having molecular weight ranging from 200 to 20,000); polyoxyethylene glycerols, polyoxyethylene sorbitols, stearoyl diacetin, and the like and mixtures thereof.

The concentration of a pharmaceutically acceptable carrier (if present in the mixture and consequently in the spreadable composition of the present invention) preferably ranges between about 10 weight percentages and about 80 weight percentages, more preferably between about 10 weight percentages and about 60 weight percentages, more preferably between about 10 weight percentages and about 40 weight percentages, more preferably between about 10 weight percentages and about 30 weight percentages, and most preferably it is about 20 weight percentages of the total weight of the composition.

According to an embodiment of the present invention, the mixture comprises at least one PAG. Preferably the PAG comprises less than about 20 weight percentages, more preferably less than about 10 weight percentages, and even more preferably less than about 1 weight percentage of the total weight of the mixture. Due to the disadvantages of using PAG in compositions as described in the Background section above, it is preferred that the mixture be substantially devoid of PAG, particularly devoid of PEG (polyethylene glycol).

Hence, according to another embodiment of the present invention, the mixture (and consequently the spreadable composition of the present invention) is substantially devoid of PAG (poly(alkylene)glycols, poly(alkoxy substituted alkylene)glycols and derivatives thereof).

As used herein, the phrase "substantially devoid of PAG" means that a composition does not include PAG or include a minor concentration of PAG, which does not cause any of the adverse side effects associated with PAG application described hereinabove. Such a concentration typically ranges between about 0.1 and 20 weight percentages, preferably between about 0.1 and 10 weight percentages, more preferably between about 0.1 and 5 weight percentages and more preferably between about 0.1 and 1 weight percentages of the total weight of the composition.

According to an embodiment of the present invention, the mixture (and consequently the spreadable composition of the present invention) further comprises a solvent. Preferably, such a solvent is selected from the group consisting of ethanol, n-propanol, isopropanol and mixtures thereof.

In a preferred embodiment, the process of the present invention also includes adding to the mixture (and consequently the spreadable composition of the present invention) an additional component or additional components that are suitable for rendering the composition of the present invention more cosmetically or aesthetically acceptable or to provide the composition with additional usage benefits.

The addition of such a component and/or of the carrier, solvent or PAG described hereinabove, can be done at any stage although it is generally most simple to add additional components before or during the heating of the mixture.

Additional components include any pharmaceutically acceptable or cosmetically acceptable ingredients such as those found in the CTFA International Cosmetic Ingredient Dictionary and Handbook, 8th edition, edited by Wenninger and Canterbery (The Cosmetic, Toiletry, and Fragrance Association, Inc., Washington, D.C., 2000).

The total concentration of the one or more additional components (if present in the mixture and consequently in the spreadable composition of the present invention) preferably ranges between about 1 weight percentage and about 20 weight percentages, more preferably between about 5 weight percentages and about 15 weight percentages, more preferably between about 5 weight percentages and about 10 weight percentages and most preferably it is about 8 weight percentages of the total weight of the composition.

Representative examples of additional components include but are not limited to anti-dandruff agents, anti-irritants, anti-oxidants, antiperspirant agents, buffering agents, bulking agents, chelating agents, colorants, deodorants, dermatological active ingredients, diluents, emollients, emulsifiers, hair conditioners, humectants, occlusive agents, oils, penetration enhancers, skin penetration enhancers, perfuming agents, permeation enhancers, pH adjusting agents, preservatives, protectants, softeners, solubilizers, stearically stabilizing substances, sunscreening agents, sun blocking agents, sunless tanning agents, surfactants and vitamins.

Suitable anti-irritants include but are not limited to steroidal and non steroidal anti-inflammatory agents, as is detailed hereinbelow, or other materials such as aloe vera, chamomile, alpha-bisabolol, cola nitida extract, green tea extract, tea tree oil, licoric extract, allantoin, caffeine or other xanthines and glycyrrhizic acid. Also suitable are derivatives, esters, salts and combinations of the same.

Suitable surfactants include but are not limited to propylene glycol mono stearate, ethoxylated esters of sorbitan fatty acids, block polymers and block copolymers (such as poloxamers and poloxamines), polyglycerol ethers and polyglycerol esters, lecithins of various origins (such as egg lecithin or soya lecithin), chemically modified lecithins (such as hydrogenated lecithins), as well as phospholipids and sphingolipids, mixtures of lecithins with phospholipids, sterols (such as cholesterol and its derivatives, specifically stigmasterol), esters and ethers of sugars or of sugar alcohols with fatty acids or fatty alcohols (such as saccharose monostearate). Also suitable are derivatives, esters, salts and combinations of the same.

In a preferred embodiment of the present invention, the mixture (and consequently the spreadable composition of the present invention) further comprises propylene glycol stearate as a surfactant.

Suitable humectants include but are not limited to guanidine, urea, glycolic acid and glycolate salts (e.g. ammonium and quaternary alkyl ammonium), lactic acid and lactate salts (e.g. ammonium and quaternary alkyl ammonium), aloe vera in any of its variety of forms (e.g., aloe vera gel), allantoin, urazole, polyhydroxy alcohols such as sorbitol, glycerol, hexanetriol, propylene glycol, butylene glycol, hexylene glycol and the like, polyethylene glycols, sugars and starches, sugar and starch derivatives (e.g., alkoxylated glucose), hyaluronic acid, lactamide monoethanolamine and acetamide monoethanolamine. Also suitable are derivatives, esters, salts and combinations of the same.

Suitable pH-adjusting agents include but are not limited to adipic acids, glycines, citric acids, calcium hydroxides, magnesium aluminometasilicates and buffers. Also suitable are derivatives, salts and combinations of the same.

Suitable chelating agents include but are not limited to ethylenediaminetetraacetic acid (EDTA). Also suitable are derivatives, esters, salts and combinations of the same.

Suitable preservatives include but are not limited to alkanols, disodium EDTA (ethylenediamine tetraacetate), EDTA salts, EDTA fatty acid conjugates, isothiazolinone, parabens such as methylparaben and propylparaben, propylene glycols, sorbates and urea derivatives such as diazolindinyl urea. Also suitable are derivatives, esters, salts and combinations of the same.

Suitable emulsifiers include but are not limited to sorbitans, alkoxylated fatty alcohols, alkylpolyglycosides, soaps, alkyl sulfates, monoalkyl and dialkyl phosphates, alkyl sulphonates and acyl isothionates. Also suitable are derivatives, esters, salts and combinations of the same.

Suitable emollients include but are not limited to dodecane, squalane, cholesterol, isohexadecane, isononyl isononanoate, PPG Ethers, petrolatum, lanolin, safflower oil, castor oil, coconut oil, cottonseed oil, palm kernel oil, palm oil, peanut oil, soybean oil and polyol carboxylic acid esters. Also suitable are derivatives, esters, salts and combinations of the same.

Suitable solubilizers that can be used include, but are not limited to, complex-forming solubilizers such as citric acid, ethylenediamine-tetraacetate, sodium metaphosphate, succinic acid, urea, cyclodextrin, polyvinylpyrrolidone, diethylammonium-ortho-benzoate, and micelle-forming solubilizers such as TWEENS and spans, e.g., TWEEN 80. Other solubilizers that are usable for the compositions of the present invention are, for example, polyoxyethylene sorbitan fatty acid ester, polyoxyethylene n-alkyl ethers, n-alkyl amine n-oxides, poloxamers, organic solvents, phospholipids and cyclodextrines. Also suitable are derivatives, esters, salts and combinations of the same.

Suitable permeation enhancers include but are not limited to dimethylsulfoxide (DMSO), dimethyl formamide (DMF), allantoin, urazole, N,N-dimethylacetamide (DMA), decylmethylsulfoxide (C₁₀ MSO), polyethylene glycol monolaurate (PEGML), propylene glycol (PG), propylene glycol monolaurate (PGML), glycerol monolaurate (GML), lecithin, the 1-substituted azacycloheptan-2-ones, particularly 1-n-dodecylcyclazacycloheptan-2-one (available under the trademark Azone™ from Whitby Research Incorporated, Richmond, Va.), alcohols, and the like. The permeation enhancer may also be a vegetable oil. Such oils include, for example, safflower oil, cottonseed oil, corn oil and arachis oil. Also suitable are derivatives, esters, salts and combinations of the same.

Suitable stearically stabilizing substances include but are not limited to poloxamers and poloxamines (block copolymers of polyoxyethylene and polyoxypropylene), ethoxylated esters of sorbitan fatty acids, in particular polysorbates (such as polysorbate 80 or Tween 80), ethoxylated mono- and diglycerides, ethoxylated lipids, ethoxylated fatty alcohols or fatty acids, and charged ionic stabilizers and peptizing agents, such as diacetyl phosphates, phosphatidyl glycerol, as well as saturated and unsaturated fatty acids, sodium cholate, sodium glycocholate, sodium taurocholate or mixtures thereof, aminoacids or peptizing agents such as sodium citrate (Lucks Int. J. Pharmaceutics 1990 63: 183-188). Also suitable are derivatives, esters, salts and combinations of the same.

Suitable dermatological inactive ingredients include but are not limited to jojoba oil, aromatic oils, methyl salicylate, wintergreen, peppermint oil, bay oil, eucalyptus oil, citrus oils, ammonium phenolsulfonate, bismuth subgallate, zinc phenolsulfonate, and zinc salicylate. Also suitable are derivatives, esters, salts and combinations of the same.

Suitable occlusive agents that can be used in preparing a composition of the present invention include, but are not limited to, petrolatum, mineral oil, beeswax, silicone oil, lanolin and oil-soluble lanolin derivatives, saturated and unsaturated fatty alcohols such as behenyl alcohol, hydrocarbons such as squalane, and various animal and vegetable oils such as almond oil, peanut oil, wheat germ oil, linseed oil, jojoba oil, oil of apricot pits, walnuts, palm nuts, pistachio nuts, sesame seeds, rapeseed, cade oil, corn oil, peach pit oil, poppyseed oil, pine oil, castor oil, soybean oil, avocado oil, safflower oil, coconut oil, hazelnut oil, olive oil, grape seed oil and sunflower seed oil. Also suitable are derivatives, esters, salts and combinations of the same.

Suitable softeners include but are not limited to ammonium lactate, dodecane, squalane, cholesterol, isohexadecane, isononyl isononanoate, PPG Ethers, petrolatum, lanolin, safflower oil, castor oil, coconut oil, cottonseed oil, palm kernel oil, palm oil, peanut oil, soybean oil and polyol carboxylic acid esters. Also suitable are derivatives, esters, salts and combinations of the same.

Suitable deodorants include but are not limited to quaternary ammonium compounds such as cetyl-trimethylammonium bromide, cetyl pyridinium chloride, benzethonium chloride, diisobutyl phenoxy ethoxy ethyl dimethyl benzyl ammonium chloride, sodium N-lauryl sarcosine, sodium N-palmlthyl sarcosine, lauroyl sarcosine, N-myristoyl glycine, potassium N-lauryl sarcosine, stearyl, trimethyl ammonium chloride, sodium aluminum chlorohydroxy lactate, tricetylmethyl ammonium chloride, 2,4,4'-trichlorio-2'-hydroxy diphenyl ether, diaminoalkyl amides such as L-lysine hexadecyl amide, heavy metal salts of citrate, salicylate, and piroctose, especially zinc salts, and acids thereof, heavy metal salts of pyrithione, especially zinc pyrithione and zinc phenolsulfate. Other deodorant actives include odor absorbing materials such as carbonate and bicarbonate salts, e.g. as the alkali metal carbonates and bicarbonates, ammonium and tetraalkylammonium carbonates and bicarbonates, especially the sodium and potassium salts. Also suitable are derivatives, esters, salts and combinations of the same.

Suitable antiperspirants include but are not limited to aluminum or zirconium astringent salts or complexes. Also suitable are derivatives, esters, and combinations of the same.

Suitable sunscreening agents and/or sun blocking agents include but are not limited to p-aminobenzoic acid, its salts and its derivatives (ethyl, isobutyl, glyceryl esters; p-dimethylaminobenzoic acid); anthranilates (i.e., o-amino-benzoates; methyl, menthyl, phenyl, benzyl, phenylethyl, linalyl, terpinyl, and cyclohexenyl esters); salicylates (amyl, phenyl, octyl, benzyl, menthyl, glyceryl, and di-pro-pyleneglycol esters); cinnamic acid derivatives (menthyl and benzyl esters, a-phenyl cinnamonitrile; butyl cinnamoyl pyruvate); dihydroxycinnamic acid derivatives (umbelliferone, methylumbelliferone, methylaceto-umbelliferone); trihydroxy-cinnamic acid derivatives (esculetin, methylesculetin, daphnetin, and the glucosides, esculin and daphnin); hydrocarbons (diphenylbutadiene, stilbene); dibenzalacetone and benzalacetophenone; naphtholsulfonates (sodium salts of 2-naphthol-3,6-disulfonic and of 2-naphthol-6,8-disulfonic acids); di-hydroxynaphthoic acid and its salts; o- and p-hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenylquinoline); hydroxy- or methoxy-substituted benzophenones; uric and violuric acids; tannic acid and its derivatives (e.g., hexaethylether); (butyl carbotol) (6-propyl piperonyl) ether; hydroquinone; benzophenones (oxybenzene, sulisobenzone, dioxybenzone, benzoresorcinol, 2,2',4,4'-tetrahydroxybenzophenone, 2,2'-dihydroxy-4,4'-dimethoxybenzophenone, octabenzone; 4-isopropyldibenzoylmethane; butylmethoxydibenzoylmethane; etocrylene; octocrylene; [3-(4'-methylbenzylidene bornan-2-one) and 4-isopropyl-di-benzoylmethane. Also suitable are derivatives, esters, salts and combinations of the same.

Suitable sunless-tanning agents include but are not limited to dihydroxyacetone, glyceraldehydes and indoles. Also suitable are derivatives, esters, salts and combinations of the same.

Suitable hair conditioners include but are not limited to collagens, cationic surfactants, modified silicones, proteins, keratins, dimethicone polyols, quaternary ammonium compounds, halogenated quaternary ammonium compounds, alkoxylated carboxylic acids, alkoxylated alcohols, alkoxylated amides, sorbitan derivatives, esters, polymeric ethers and glyceryl esters. Also suitable are derivatives, esters, salts and combinations of the same.

Suitable anti-dandruff agents include but are not limited to zinc pyrithione, shale oil and derivatives thereof such as sulfonated shale oil, selenium sulfide, sulfur; salicylic acid, coal tar, povidone-iodine, imidazoles such as ketoconazole, dichlorophenyl imidazolodioxalan, clotrimazole, itraconazole, miconazole, climbazole, tioconazole, sulconazole, butoconazole, fluconazole, miconazolenitrite and any possible stereo isomers and derivatives thereof such as anthralin, piroctone olamine (Octopirox), selenium sulfide, and ciclopirox olamine. Also suitable are derivatives, esters, salts and combinations of the same.

Suitable antioxidants include but are not limited to ascorbic acid (vitamin C) and its salts, ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Trolox^{R}), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, methionine, proline, superoxide dismutase, silymarin, tea extracts, grape skin/seed extracts, melanin, and rosemary extracts. Also suitable are derivatives, esters, salts and combinations of the same.

Suitable vitamins that can be used include, but are not limited to, vitamin A and its analogs and derivatives: retinol, retinal, retinyl palmitate, retinoic acid, tretinoin, iso-tretinoin (known collectively as retinoids), vitamin E (tocopherol and its derivatives), vitamin C (L-ascorbic acid and its esters and other derivatives), vitamin B₃ (niacinamide and its derivatives), alpha hydroxy acids (such as glycolic acid, lactic acid, tartaric acid, malic acid, citric acid, etc.) and beta hydroxy acids (such as salicylic acid and the like). Also suitable are derivatives, esters, salts and combinations of the same.

According to an embodiment of the present invention, the mixture (and consequently the spreadable composition of the present invention) further comprises at least one active ingredient (also called "active"). The active ingredient can be a pharmaceutically, cosmetically and/or cosmeceutically active ingredient. Incorporation of such active ingredients renders the spreadable composition of the present invention a pharmaceutical, cosmetic and/or cosmeceutical composition.

Representative examples of active ingredients that can be incorporated within the spreadable composition of the present invention include, without limitation, antibiotic agents, antimicrobial agents, anti-acne agents, antibacterial agents, antifungal agents, antiviral agents, steroidal and non steroidal anti-inflammatory agents (NSAIDS), anesthetic agents, antipruriginous agents, antiprotozoal agents, anti-oxidant agents, chemotherapeutic agents, antidepressant agents, hormones and antidandruff agents, as well as cosmetic active ingredients such as, but not limited to, sunscreening or blocking agents, dyes, hair conditioning agents, cleansing agents, deodorants, sunless tanning agents and the like, or any combination thereof.

Suitable anti-acne agents include but are not limited to keratolytics such as salicylic acid, sulfur, glycolic, pyruvic acid, resorcinol, and N-acetylcysteine; retinoids such as retinoic acid and its derivatives (e.g., cis and trans, esters); antibiotics and antimicrobials such as mupirocin, benzoyl peroxide, octopirox, erythromycin, zinc, tetracyclin, triclosan, azelaic acid and its derivatives, phenoxy ethanol and phenoxy proponol, ethylacetate, clindamycin and meclocycline; sebostats such as flavinoids; alpha and beta hydroxy acids; and bile salts such as scymnol sulfate and its derivatives, deoxycholate, and cholate. Also suitable are derivatives, esters, salts and combinations of the same.

Suitable NSAIDS include but are not limited to oxicams, such as piroxicam, isoxicam, tenoxicam, sudoxicam, and CP-14,304. Salicylates, such as aspirin, disalcid, benorylate, trilisate, safapryn, solprin, diflunisal, and fendosal. Acetic acid derivatives, such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac. Fenamates, such as mefenamic, meclofenamic, flufenamic, niflumic, and tolfenamic acids. Propionic acid derivatives, such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic. Pyrazoles, such as phenylbutazone, oxyphenbutazone, feprazone, azapropazone, and trimethazone. Also suitable are derivatives, esters, salts and combinations of the same. For example, etofenamate, a flufenamic acid derivative, is particularly useful for topical application.

Suitable anti-inflammatory agents include but are not limited to corticosteroids such as hydrocortisone, hydroxyltriamcinolone, alpha-methyl dexamethasone, dexamethasone-phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, diflurosone diacetate, fluradrenolone acetonide, medrysone, amcinafel, amcinafide, betamethasone and the balance of its esters, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate and triamcinolone. Also suitable are derivatives, esters, salts and combinations of the same.

Suitable antipruritic agents include but are not limited to methdilazine, trimeprazine. Also suitable are derivatives, esters, salts and combinations of the same.

Suitable anesthetic agents include but are not limited to lidocaine, bupivacaine, chlorprocaine, dibucaine, etidocaine, mepivacaine, tetracaine, dyclonine, hexylcaine, procaine, cocaine, ketamine, pramoxine and phenol. Also suitable are derivatives, esters, salts and combinations of the same.

Suitable chemotherapeutic agents include but are not limited to daunorubicin, doxorubicin, idarubicin, amrubicin, pirarubicin, epirubicin, mitoxantrone, etoposide, teniposide, vinblastine, vincristine, mitomycin C, 5-FU, paclitaxel, docetaxel, actinomycin D, colchicine, topotecan, irinotecan, gemcitabine cyclosporin, verapamil, valspodor, probenecid, MK571, GF120918, LY335979, biricodar, terfenadine, quinidine, pervilleine A, and XR9576. Also suitable are derivatives, esters, salts and combinations of the same.

Suitable antidepressant agents include but are not limited to classes of antidepressant agents such as norepinephrine-reuptake inhibitors ("NRIs"), selective-serotonin-reuptake inhibitors (SSRIs), monoamine-oxidase inhibitors (MAOIs), serotonin-and-noradrenaline-reuptake inhibitors ("SNFIs), corticotropin-releasing factor (CRF) antagonists, α-adrenoreceptor antagonists, NK1-receptor antagonists, 5-HT_{1A}-receptor agonist, antagonists, and partial agonists, atypical antidepressants, and other antidepressants. Examples of suitable norepinephrine-reuptake inhibitors include, but are not limited to amitriptyline, desmethylamitriptyline, clomipramine, doxepin, imipramine, imipramine-oxide, trimipramine; adinazolam, amiltriptylinoxide, amoxapine, desipramine, maprotiline, nortriptyline, protriptyline, amineptine, butriptyline, demexiptiline, dibenzepin, dimetacrine, dothiepin, fluacizine, iprindole, lofepramine, melitracen, metapramine, norclolipramine, noxiptilin, opipramol, perlapine, pizotyline, propizepine, quinupramine, reboxetine, tianeptine, and pharmaceutically acceptable salts thereof. Examples of suitable serotonin-reuptake inhibitors include, but are not limited to, binedaline, m-chloropiperzine, citalopram, duloxetine, etoperidone, femoxetine, fluoxetine, fluvoxamine, indalpine, indeloxazine, milnacipran, nefazodone, oxaflazone, paroxetine, prolintane, ritanserin, sertraline, tandospirone, venlafaxine and zimeldine. Also suitable are derivatives, esters, salts and combinations of the same.

Suitable hormones include, for example, androgenic compounds and progestin compounds.

Suitable androgenic compounds include, but are not limited to, methyltestosterone, androsterone, androsterone acetate, androsterone propionate, androsterone benzoate, androsteronediol, androsteronediol-3-acetate, androsteronediol-17-acetate, androsteronediol 3-17-diacetate, androsteronediol-17-benzoate, androsteronedione, androstenedione, androstenediol, dehydroepiandrosterone, sodium dehydroepiandrosterone sulfate, dromostanolone, dromostanolone propionate, ethylestrenol, fluoxymesterone, nandrolone phenpropionate, nandrolone decanoate, nandrolone furylpropionate, nandrolone cyclohexane-propionate, nandrolone benzoate, nandrolone cyclohexanecarboxylate, androsteronediol-3-acetate-1-7-benzoate, oxandrolone, oxymetholone, stanozolol, testosterone, testosterone decanoate, 4-dihydrotestosterone, 5α-dihydrotestosterone, testolactone, 17α-methyl-19-nortestosterone and pharmaceutically acceptable esters and salts thereof, and combinations of any of the foregoing.

Suitable progestin compounds include, but are not limited to, desogestrel, dydrogesterone, ethynodiol diacetate, medroxyprogesterone, levonorgestrel, medroxyprogesterone acetate, hydroxyprogesterone caproate, norethindrone, norethindrone acetate, norethynodrel, allylestrenol, 19-nortestosterone, lynoestrenol, quingestanol acetate, medrogestone, norgestrienone, dimethisterone, ethisterone, cyproterone acetate, chlormadinone acetate, megestrol acetate, norgestimate, norgestrel, desogrestrel, trimegestone, gestodene, nomegestrol acetate, progesterone, 5α-pregnan-3β,20α-diol sulfate, 5α-pregnan-3β,20β-diol sulfate, 5α-pregnan-3β.-ol-20-one, 16,5α-pregnen-3β-ol-20-one, 4-pregnen-20β-ol-3-one-20-sulfate, acetoxypregnenolone, anagestone acetate, cyproterone, dihydrogesterone, flurogestone acetate, gestadene, hydroxyprogesterone acetate, hydroxymethylprogesterone, hydroxymethyl progesterone acetate, 3-ketodesogestrel, megestrol, melengestrol acetate, norethisterone and mixtures thereof.

The at least one active ingredient added to a mixture (and that is consequently found in a composition of the present invention) also includes but is not limited to antimicrobial agents, antibacterial agents, antifungal agents, antiprotozoal agents, antiviral agents, beta-lactam drugs, quinolone drugs, ciprofloxacin, norfloxacin, tetracycline, erythromycin, amikacin, triclosan, doxycycline, capreomycin, chlorhexidine, chlortetracycline, oxytetracycline, clindamycin, ethambutol, metronidazole, pentamidine, gentamicin, kanamycin, lineomycin, methacycline, methenamine, minocycline, neomycin, netilmicin, streptomycin, tobramycin, miconazole, tetracycline hydrochloride, famesol, erythromycin estolate, erythromycin stearate, amikacin sulfate, doxycycline hydrochloride, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amanfadine sulfate, triclosan, octopirox, parachlorometa xylenol, nystatin, tolnaftate and clotrimazole. Also suitable are derivatives, esters, salts and combinations of the same.

The total concentration of the active ingredients (if present in the mixture and consequently in the spreadable composition of the present invention) preferably ranges between about 0.1 weight percentage and about 10 weight percentages, more preferably between about 0.5 weight percentages and about 5 weight percentages, more preferably between about 1 weight percentages and about 5 weight percentages and most preferably it is about 2 weight percentages of the total weight of the composition.

As stated above, according to the teachings of the present invention there is also provided a spreadable composition, which is based on one or more hard fats as described hereinabove. Preferably, the spreadable composition of the present invention is substantially prepared according to the process of the present invention, as described herein.

The spreadable composition of the present invention can simply serve as a base, which can be mixed or incorporated within other compositions.

When the spreadable composition of the present invention includes an active ingredient, as described hereinabove, the spreadable composition of the present invention is a pharmaceutical, cosmetic or cosmeceutic composition.

Such a pharmaceutical, cosmetic or cosmeceutic composition, according to a feature of the present invention, is packaged in a packaging material and identified in print, in or on the packaging material, for use for a need selected from the group consisting of curing a condition, treating a condition, preventing a condition, treating symptoms of a condition, curing symptoms of a condition, ameliorating symptoms of a condition, treating effects of a condition, ameliorating effects of a condition, and preventing results of a condition for which the composition is beneficial.

According to a feature of the present invention, and depending on the components making up a composition of the present invention, the condition identified can be, for example, eye disorders, ear disorders, mucosal membrane disorders, and skin and/or scalp disorders, such as, for example, actinic keratoses, eczema, dermatitis, BCC (basal cell carcinoma), superficial BCC, Bowen's disease, chronic superficial scaly, dermatosis, parapsoriasis, cradle cap, cutaneous T-cell lymphoma, Darier's disease, disseminated superficial, actinic porokeratosis, dry skin, erythrasma, exfoliative keratolysis, Grover's disease, ichthyosis, juvenile plantar dermatosis, lichen striatus, lupus erythematosus, pellagra, pityriasis alba, pityriasis lichenoides, pityriasis rosea, pityriasis rubra pilaris, pityriasis versicolor, psoriasis, Reiter's syndrome, seborrhoeic dermatitis, impetigo, wounds, bums, and infections such as fungal infections, gram-positive aerobe infections, gram-negative aerobe infections, rectal infections and vaginal infections.

The packaged composition can further be identified for use for a need such as, for example, hair conditioning, sunscreening, make-up, hair coloring or any other cosmetic or cosmeceutical need.

According to a feature of the present invention the spreadable composition is made in a form and appropriately packaged as a cream, gel, lotion, ointment, paste, pledget or pad. Due to its inherent characteristics, the composition is preferably in a form of an ointment.

The packaging is preferably performed during the preparation process, before re-cooling the mixture, as is detailed hereinabove.

The package or receptacle in which the compositions of the present invention is packed preferably comprises a tube (such as a squeeze tube), a jar, a bottle (for example with a pump dispenser), a unit does or a pressurized container, using techniques well known to those skilled in the art and as set forth in reference works such as Remington's Pharmaceutical Science 15^{th} Ed. It is preferred that the package or receptacle is such that, during use, contact of the unused compositions with the environment is minimized.

A spreadable composition prepared according to the process described herein is useful in implementing a method of treatment, especially when the composition includes a pharmaceutically active compound.

Hence, according to the teachings of the present invention there is also provided a method of treatment comprising administering an effective amount of the spreadable composition of the present invention (especially when the composition includes an active ingredient) to a mammal (human or non-human) in need thereof. Administration of a composition of the present invention is preferably topical administration to ears, eyes, skin and/or mucous membranes, but can also be effected buccally, dermally, intranasally, lingually, mucosally, rectally, sublingually, urethrally and vaginally.

The term "effective amount" describes an amount of a composition that is sufficient to significantly induce a positive modification in the condition being treated, but low enough to avoid significant side effects, within the scope of sound judgment of one skilled in the art. The effective amount of the composition may vary with the particular area being treated, the age and physical condition of the mammal being treated, the severity of the condition, the duration of the treatment, the nature of concurrent therapy, the specific compound, composition or other material employed, the particular carrier utilized, and like factors within the knowledge and expertise of one skilled in the art.

The need for which a spreadable composition of the present invention is used is generally a need arising from a condition in which treatment by the pharmaceutically, cosmetically or cosmeceutically active ingredient is beneficial, as is detailed hereinabove. Such a need includes, for example, curing the condition, treating the condition, preventing the condition, treating symptoms of the condition, curing symptoms of the condition, ameliorating symptoms of the condition, treating effects of the condition, ameliorating effects of the condition, and preventing results of the condition, whereby the condition can be any of the conditions described hereinabove.

Additional objects, advantages, and novel features of the present invention will become apparent to one ordinarily skilled in the art upon examination of the following non-limiting, example. Additionally, each of the various embodiments and aspects of the present invention as delineated hereinabove and as claimed in the claims section below finds experimental support in the following examples.

### EXAMPLES

Reference is now made to the following examples, which together with the above description illustrate the invention in a non-limiting fashion.

### MATERIALS AND EXPERIMENTAL METHODS

### Materials:

Reagents were purchased from various commercial suppliers.

The hard fat used (caprylic/capric/stearic triglyceride), was purchased from SASOL, under the name Sofistan 378.

### Experimental methods:

Semi-industrial scale syntheses were performed in a Scott Turbon Mixer having a capacity of 20 liters.

Viscosity of compositions was measured using a Brookfield Voscometer RVDV -1+1.

### EXAMPLE 1

### SMALL SCALE PREPARATION OF A SPREADABLE COMPOSITION

70 grams hard fat (caprylic/capric/stearic triglyceride), 15 grams castor oil, 8 grams propylene glycol stearate, 5 grams oleyl alcohol and 2 grams Mupirocin were placed in a 100 ml glass flask fitted with a thermometer, a mechanical stirrer and immersed in an oil bath. The oil bath was heated to 75 °C and the mixture stirred for 30 minutes. The flask was removed from the oil and the mixture allowed to cool while stirring. The mixture was observed to congeal at a temperature of about 25 °C. The flask containing the mixture was then immersed in a 32 °C oil bath and stirred for five hours. The flask was removed from the bath, the resulting molten mixture was poured in a jar and was allowed to cool to room temperature. The congealed product was labeled product I.

### EXAMPLE 2

### SEMI-INDUSTRIAL PREPARATION OF SPREADABLE COMPOSITIONS

7000 grams hard fat (caprylic/capric/stearic triglyceride), 1500 grams castor oil, 800 grams propylene glycol stearate, 500 grams oleyl alcohol and 200 grams Mupirocin were placed in a semi-industrial scale reactor. The mixture was heated to 75 °C and was stirred for 30 minutes. The mixture was allowed to cool in the reactor to 25 °C, while stirring. The mixture was stirred at 25 °C for a period of 60 minutes. The mixture was then re-heated to 32 °C oil bath and was stirred for five hours. While stirring, a 15 grams aluminum squeeze-tube was filled with the mixture and was thereafter allowed to cool to room temperature. The above procedure was repeated 4 times. Each of the squeeze-tubes was labeled as containing product II, III, IV or V.

### EXAMPLE 3

### VISCOSITY

After 1 day the viscosities of products I -V were measured and found to be 4 Poise at 20 °C. After 6 months storage at 20 °C, the viscosities of products I-V were measured and found to be unchanged.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A spreadable composition comprising at least one hard fat.

2. The spreadable composition of claim 1, being occlusive, creamy, non-greasy, smooth and easy to spread.

3. The spreadable composition of claim 2, having a viscosity that ranges between 2 Poise and about 2500 Poise at a temperature of about 20 °C, preferably between 3 and 2000 Poise.

4. The spreadable composition of claim 1, being substantially devoid of PAG.

5. The spreadable composition of claim 1, further comprising a carrier.

6. The spreadable composition of claim 5, wherein said carrier is selected from the group consisting of water, a glycol, a polyalkylene glycol, an ester, an amide, a protein hydrolysate, an alkylated protein hydrolysate, a lanolin or a derivative thereof, a monohydric alcohol, a polyhydric alcohol, an ether, a carbowax, a polyoxyethylene glycerol, a polyoxyethylene sorbitol, a stearoyl diacetin and any combination thereof.

7. The spreadable composition of claim 1, further comprising at least one polyalkylene glycol (PAG).

8. The spreadable composition of claim 7, wherein a concentration of said PAG is less than about 20 weight percentages of the total weight of the composition, preferably less than about 10 weight percentages and more preferably 1 weight percentage.

9. The spreadable composition of claim 1, further comprising propylene glycol stearate.

10. The spreadable composition of claim 1, further comprising at least one additional component selected from the group consisting of an anti-dandruff agent, an anti-irritant, an anti-oxidant, an antiperspirant agent, a buffering agent, a bulking agent, a chelating agent, a colorant, a deodorant, a dermatological active ingredient, a diluent, an emollient, an emulsifier, a hair conditioner, a humectant, an occlusive agent, oil, a penetration enhancer, a skin penetration enhancer, a perfuming agent, a permeation enhancer, a pH adjusting agent, a preservative, a protectant, a softener, a solubilizer, a stearically stabilizing substance, a sunscreening agent, a sun blocking agent, a sunless tanning agent, a surfactant and a vitamin.

11. The spreadable composition of claim 1-10, further comprising at least one pharmaceutically, cosmetically or cosmeceutically active ingredient.

12. The spreadable composition of claim 11, wherein said at least on pharmaceutically, cosmetically or cosmeceutically active ingredient is selected from the group consisting of an antibiotic agent, an antimicrobial agent, an anti-acne agent, an antibacterial agent, an antifungal agent, an antiviral agent, an anti-inflammatory agent, an anesthetic agent, an antipruriginous agent, an antiprotozoal agent, an anti-oxidant, a chemotherapeutic agent, an antidepressant agent, a hormone, an antidandruff agent, a sunscreening agent, a sun blocking agent, a sunless tanning agent, a dye, a deodorant, a hair conditioning agent and a cleansing agent.

13. The spreadable composition of claim 11, packaged in a packaging material and identified in print, in or on said packaging material, for use for a need selected from the group consisting of curing a condition, treating a condition, preventing a condition, treating symptoms of a condition, curing symptoms of a condition, ameliorating symptoms of a condition, treating effects of a condition, ameliorating effects of a condition, and preventing results of a condition.

14. The spreadable composition of claim 13, wherein said condition is selected from the group consisting of a medical condition, a cosmetic condition and a cosmeceutical condition.

15. The spreadable composition of claim 12, wherein said medical condition is selected from the group consisting of an eye disorder, an ear disorder, a mucosal membrane disorder and a skin and/or scalp disease or disorder.

16. The spreadable composition of claim 15, wherein said skin and/or scalp disease or disorder is selected from the group consisting of actinic keratoses, eczema, dermatitis, BCC, superficial BCC, Bowen's disease, chronic superficial scaly, dermatosis, parapsoriasis, cradle cap, cutaneous T-cell lymphoma, Darier's disease, disseminated superficial, actinic porokeratosis, dry skin, erythrasma, exfoliative keratolysis, an infection, a fungal infection, Grover's disease, ichthyosis, juvenile plantar dermatosis, lichen striatus, lupus erythematosus, pellagra, pityriasis alba, pityriasis lichenoides, pityriasis rosea, pityriasis rubra pilaris, pityriasis versicolor, psoriasis, Reiter's syndrome, seborrhoeic dermatitis, a tinea infection, impetigo, a wound and a bum.

17. The spreadable composition of claim 1, wherein a concentration of said at least one hard fat ranges between about 20 weight percentages and 100 weight percentages of the total weight of the composition, preferably 20 to 80 weight percentages.

18. The spreadable composition of claim 1, wherein said at least one hard fat comprises a triglyceride.

19. The spreadable composition of claim 18, wherein said triglyceride is a mixed triester of glycerin with caprylic, capric and stearic acids.

20. A process of preparing a spreadable composition for topical application, the process comprising:
providing a mixture including at least one hard fat;
heating said mixture to a first temperature, said first temperature being higher than a congelation temperature of said mixture;
cooling said mixture to a second temperature;
re-heating said mixture to a third temperature, said third temperature being higher than said congelation temperature of said mixture; and
re-cooling said mixture to an ambient temperature, thereby obtaining the spreadable composition.

21. The process of claim 20, wherein said second temperature is lower than said congelation temperature of said mixture.

22. The process of claim 20, wherein said heating is performed while stirring said mixture.

23. The process of claim 20, wherein said cooling is performed while stirring said mixture.

24. The process of claim 20, wherein said re-heating is performed while stirring said mixture.

25. The process of claim 20, wherein said first temperature ranges between about 40 °C and about 100 °C, preferably 50°C to 80°C.

26. The process of claim 20, wherein said second temperature ranges between about 10°C and about 30°C, preferably between 18°C and 28°C.

27. The process of claim 20, wherein said third temperature ranges between about 30 °C and about 40 °C, preferably 30°C to 35°C.

28. The process of claim 20, wherein said mixture is maintained at said third temperature for a period of time that ranges between about 180 minutes and about 600 minutes, preferably about 300 minutes.

29. The process of claim 20, further comprising, subsequent to said re-heating and prior to said re-cooling, filling a receptacle with said mixture.

30. The process of claim 29, wherein during said filling a temperature of said mixture is substantially maintained at a fourth temperature.

31. The process of claim 30, wherein said fourth temperature ranges between about 20 °C and about 40 °C, preferably about 30°C and 35°C.

32. The process of claim 20, wherein the spreadable composition has a viscosity that ranges between about 2 Poise and about 2500 Poise at a temperature of about 20 °C, preferably 3 Poise and 2000 Poise..

33. The process of claim 20, wherein a concentration of said at least one hard fat ranges between about 20 weight percentages and about 100 weight percentages of the total weight of said mixture, preferably between 50 weight percentages and 100 weight percentages.

34. The process of claim 20, wherein said at least one hard fat comprises a triglyceride.

35. The process of claim 34, wherein said triglyceride is a mixed triester of glycerin with caprylic, capric and stearic acids.

36. The process of claim 20, wherein said mixture further comprises a carrier.

37. The process of claim 36, wherein said carrier comprises castor oil and/or oleyl alcohol.

38. The process of claim 36, wherein said carrier is selected from the group consisting of water, a glycol, a polyalkylene glycol, an ester, an amide, a protein hydrolysate, an alkylated protein hydrolysate, a lanolin or a derivative thereof, a monohydric alcohol, a polyhydric alcohol, an ether, a carbowax, a polyoxyethylene glycerol, a polyoxyethylene sorbitol, a stearoyl diacetin and any combination thereof.

39. The process of claim 20, wherein said mixture further comprises at least one polyalkylene glycol (PAG).

40. The process of claim 39, wherein a concentration of said PAG is less than about 20 weight percentages of the total weight of said composition, preferably less than 10 weight percentages, more preferably less than 1 percentage.

41. The process of claim 20, wherein the spreadable composition is substantially devoid of PAG.

42. The process of claim 20, wherein said mixture further comprises a solvent.

43. The process of claim 42, wherein said solvent is selected from the group consisting of ethanol, n-propanol, isopropanol and mixtures thereof.

44. The process of claim 20, wherein said mixture further comprises a propylene glycol stearate.

45. The process of claim 20, wherein said mixture further comprises at least one additional component selected from the group consisting of an anti-dandruff agent, an anti-irritant, an anti-oxidant, an antiperspirant agent, a buffering agent, a bulking agent, a chelating agent, a colorant, a deodorant, a dermatological active ingredient, a diluent, an emollient, an emulsifier, a hair conditioner, a humectant, an occlusive agent, oil, a penetration enhancer, a skin penetration enhancer, a perfuming agent, a permeation enhancer, a pH adjusting agent, a preservative, a protectant, a softener, a solubilizer, a stearically stabilizing substance, a sunscreening agent, a sun blocking agent, a sunless tanning agent, a surfactant and a vitamin.

46. The process of claim 20, wherein said mixture further comprises at least one pharmaceutically, cosmetically or cosmeceutically active ingredient.

47. The process of claim 46, wherein said at least on pharmaceutically, cosmetically or cosmeceutically active ingredient is selected from the group consisting of an antibiotic agent, an antimicrobial agent, an anti-acne agent, an antibacterial agent, an antifungal agent, an antiviral agent, an anti-inflammatory agent, an anesthetic agent, an antipruriginous agent, an antiprotozoal agent, an anti-oxidant, a chemotherapeutic agent, an antidepressant agent, a hormone, an antidandruff agent, a sunscreening agent, a sun blocking agent, a sunless tanning agent, a dye, a deodorant, a hair conditioning agent and a cleansing agent.

48. A spreadable composition substantially prepared according to the process of claims 20-47.

49. The spreadable composition of claim 48, being occlusive, creamy, non-greasy, smooth and easy to spread.

50. Use of an effective amount of the spreadable composition of claim 11 for the manufacture of a medicament for administering to a mammal in need thereof.

51. The use of claim 50, wherein said mammal is a non-human mammal.

52. The use of claim 50, wherein said mammal is a human.

53. The use of claim 50, wherein said administering is effected by topically applying said spreadable composition to at least one affected area of said mammal.

54. The use of claim 53, wherein said at least one affected area is selected from the group consisting of an eye, an ear, a skin, a scalp, a mucosal membrane.

55. The use of claim 50, wherein said administering is effected in a manner selected from the group consisting of buccally, dermally, intranasally, lingually, mucosally, rectally, sublingually, topically, urethrally and vaginally.

56. The use of claim 50, wherein said at least one pharmaceutically, cosmetically or cosmeceutically active ingredient is selected from the group consisting of an antibiotic agent, an antimicrobial agent, an anti-acne agent, an antibacterial agent, an antifungal agent, an antiviral agent, an anti-inflammatory agent, an anesthetic agent, an antipruriginous agent, an antiprotozoal agent, an anti-oxidant, a chemotherapeutic agent, an antidepressant agent, and an antidandruff agent.

57. The use of claim 50, wherein said need arises from a condition in which treatment by said pharmaceutically, cosmetically or cosmeceutically active ingredient is beneficial.

58. The use of claim 57, wherein said condition is selected from the group consisting of an eye disorder, an ear disorder, a mucosal membrane disorder and a skin and/or scalp disease or disorder.

59. The use of claim 58, wherein said skin and/or scalp disease or disorder is selected from the group consisting of actinic keratoses, eczema, dermatitis, BCC, superficial BCC, Bowen's disease, chronic superficial scaly, dermatosis, parapsoriasis, cradle cap, cutaneous T-cell lymphoma, Darier's disease, disseminated superficial, actinic porokeratosis, dry skin, erythrasma, exfoliative keratolysis, fungal infections, Grover's disease, ichthyosis, juvenile plantar dermatosis, lichen striatus, lupus erythematosus, pellagra, pityriasis alba, pityriasis lichenoides, pityriasis rosea, pityriasis rubra pilaris, pityriasis versicolor, psoriasis, Reiter's syndrome, seborrhoeic dermatitis, tinea infections, impetigo, wounds, bums, infections, fungal infections, gram-positive aerobe infections, gram-negative aerobe infections, rectal infections, vaginal infections and wounds of a mucosal membrane.

60. The use of claim 50, wherein said spreadable composition has a viscosity that ranges between about 2 Poise and about 2500 Poise at a temperature of about 20 °C, preferably 2 to 2000 Poise.

61. The use of claim 50, wherein said second temperature is lower than said congelation temperature of said mixture.

62. The use of claim 50, wherein said heating is performed while stirring said mixture.

63. The use of claim 50, wherein said cooling is performed while stirring said mixture.

64. The use of claim 50, wherein said re-heating is performed while stirring said mixture.

65. The use of claim 50, wherein said first temperature ranges between about 40 °C and about 100 °C, preferably 50°C to 80°C.

66. The use of claim 50, wherein said second temperature ranges between about 10 °C and about 30 °C, preferably 18°C to 28°C.

67. The use of claim 50, wherein said third temperature ranges between about 30 °C and about 40 °C, preferably between 30°C and 35°C.

68. The use of claim 50, wherein said mixture is maintained at said third temperature for a period of time that ranges between about 180 minutes and about 600 minutes, preferably about 300 minutes

69. The use of claim 50, wherein said process further comprises, subsequent to said re-heating and prior to said re-cooling, filling a receptacle with said mixture.

70. The use of claim 69, wherein during said filling a temperature of said mixture is substantially maintained at a fourth temperature.

71. The use of claim 70, wherein said fourth temperature ranges between about 20 °C and about 40 °C, preferably between 30°C and 35°C.

72. The use of claim 50, wherein a concentration of said at least one hard fat ranges between about 20 weight percentages and about 100 weight percentages of the total weight of said mixture, preferably between 50 and 100 weight percentages.

73. The use of claim 50, wherein said at least one hard fat comprises a triglyceride.

74. The use of claim 73, wherein said triglyceride is a mixed triester of glycerin with caprylic, capric and stearic acids.

75. The use of claim 50, wherein said mixture further comprises a carrier.

76. The use of claim 75, wherein said carrier comprises castor oil and/or oleyl alcohol.

77. The use of claim 75, wherein said carrier is selected from the group consisting of water, a glycol, a polyalkylene glycol, an ester, an amide, a protein hydrolysate, an alkylated protein hydrolysate, a lanolin or a derivative thereof, a monohydric alcohol, a polyhydric alcohol, an ether, a carbowax, a polyoxyethylene glycerol, a polyoxyethylene sorbitol, a stearoyl diacetin and any combination thereof.

78. The use of claim 50, wherein said mixture further comprises at least one polyalkylene glycol (PAG).

79. The use of claim 78, wherein a concentration of said PAG is less than about 20 weight percentages of the total weight of said spreadable composition, preferably less than about 10% by weight, and more preferably less than 1% by weight.

80. The use of claim 50, wherein said spreadable composition is substantially devoid of PAG.

81. The use of claim 50, wherein said mixture further comprises a solvent.

82. The use of claim 81, wherein said solvent is selected from the group consisting of ethanol, n-propanol, isopropanol and mixtures thereof.

83. The use of claim 50, wherein said mixture further comprises a propylene glycol stearate.

84. The use of claim 50, wherein said mixture further comprises at least one additional component selected from the group consisting of an anti-dandruff agent, an anti-irritant, an anti-oxidant, an antiperspirant agent, a buffering agent, a bulking agent, a chelating agent, a colorant, a deodorant, a dermatological active ingredient, a diluent, an emollient, an emulsifier, a hair conditioner, a humectant, an occlusive agent, oil, a penetration enhancer, a skin penetration enhancer, a perfuming agent, a permeation enhancer, a pH adjusting agent, a preservative, a protectant, a softener, a solubilizer, a stearically stabilizing substance, a sunscreening agent, a sun blocking agent, a sunless tanning agent, a surfactant and a vitamin.
